# EUROPEAN PATENT SPECIFICATION

(11) **EP 1 516 003 B1**
(45) Date of publication and mention of the grant of the patent: **11.04.2007**
(21) Application number: 03733793.8
(22) Date of filing: 23.06.2003
(51) Int. Cl.: C08G 18/10, C08G 18/42, A61L 27/18

(54) **LINEAR BLOCK POLYMER**
LINEARES BLOCKPOLYMER
POLYMERE SEQUENCE LINEAIRE

(30) Priority: 20.06.2002 SE 0201948
(43) Date of publication of application: 23.03.2005
(73) Proprietor: Artimplant AB, 421 32 Västra Frölunda (SE)
(72) Inventor: GISSELFÄLT, Katrin, S-412 57 Göteborg (SE)
(74) Representative: Romare, Laila Anette
(86) International application number: PCT/SE2003/001085
(87) International publication number: WO 2004/000904

(56) References cited:
- WO-A1-00/45869
- WO-A1-97/22643
- WO-A1-99/64491
- DE-A1- 19 841 512
- US-B1- 6 221 997
- GISSELFALT KATRIN ET AL.: 'A biodegradable material for ACL reconstruction' MACROMOL. SYMP. vol. 130, 1998, pages 103 - 111, XP002962682
- LILJENSTEN ELISABETH ET AL.: 'Studies of polyurethane urea bands for ACL reconstruction' JOURNAL OF MATERIALS SCIENCE: MATERIALS IN MEDICINE vol. 13, 2002, pages 351 - 359, XP002962683
- HONG CHEN ET AL.: 'Biocompatible waterborne polyurethane using L-lysine as extender' POLYMER PREPRINTS vol. 42, no. 2, 2001, pages 660 - 661, XP002962684
- SPAANS C.J. ET AL.: 'High molecular weight polyurethanes and a polyurethane urea based on 1,4-butanediisocyanate' POLYMER BULLETIN vol. 41, 1998, pages 131 - 138, XP000774961

## Description

### TECHNICAL FIELD

The invention relates to a linear block polymer, to various preparations made from said linear block polymer, and to an implant comprising the linear block polymer.

### BACKGROUND ART

Certain injuries to soft tissue of the body do not heal by itself. One example of such an injury is injuries to the meniscus, injuries common to certain athletes. When such an injury happened, the injured part was often removed, resulting in reduced bodily functions. This often meant the end of the career of the athlete. A continued high load on a knee without a meniscus leads to wear of the skeleton on the bearing surfaces, with permanent pains as a probable outcome.

Another tissue that is often associated with the injuries of athletes is the anterior cruciate ligament (ACL), the primary and most important stabiliser of the knee. To continue with certain sports after having injured the cruciate ligaments is often associated with higher risks for skeleton damages.

Already in the 1960-ties were attempts done to replace injured ligaments with artificial implants. The materials used for the artificial ligaments were for instance polytetrafluor ethylene, polyethylene terephtalate, polypropylene, polyethylene and carbon fibers.

Unfortunately, these early implants exhibited a number of drawbacks. Among other things, irreversible elongation and rupture of the implants took place. Young's modulus for the above mentioned materials is often too high for the materials to function well as implants that are a replacement for the anterior cruciate ligament.

In addition, it was desired to have a material in the implant that was biocompatible and biodegradable in that sense that it stimulated growth in the injured tissue while itself degraded. Thus, making the injured tissue re-growth and taking over the bodily function as the implant deteriorated. The above mentioned materials did not fulfil this criteria.

In SE, 505 703, C2, a material for use in implants disclosed, that is biocompatible and biodegradable. The material disclosed is a linear block polymer comprising urea and urethane groups, which polymer exhibits a molecular weight of at least 10⁴ Dalton.

While an implant comprising the described material has functioned satisfactorily, there are numerous biological and mechanical parameters that are to be fulfilled by a material for use in a biodegradable implant.

High initial strength of the implant is required to prevent rupture of the implant before the bodily tissue has been able to re-growth and take over the bodily function. A stepwise degradation of the material is essential to induce re-growth of the bodily tissue. Degradation speed should be balanced to get optimum re-growth of tissue. The mechanical properties of the implant should be corresponding to that of the bodily tissue it is replacing so as to achieve as normal bodily function as possible during healing.

Thus, there still exists a need to optimise the mechanical properties and the degradation speed for a material suitable for use in an implant.

### DISCLOSURE OF INVENTION

The above problems are solved by the present invention through the manufacturing of a linear block polymer according to Formula (1): 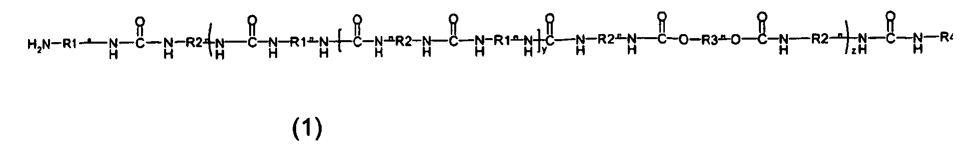 wherein
R1 is derived from a diamine, e.g. ethylene diamine, 1,2-diamino propane or 1,3-diamino propane;
R2 is derived from an aromatic diisocyanate;
R3 is derived from an esterdiol;
R4 is derived from dibutyl amine or ethanolamine;
Where 0 < y < 4 and z > 8,
the monomers from which R2 and R3 are derived from are added in such amounts that the molar ratio between R2 and R3 is larger than 2:1. That is, the number of molars of monomer added that R2 is derived from, is more than twice the number of molars of monomer added that R3 is derived from. As described in the examples below, more than twice the amount of isocyanate to that of esterdiol is added during the polymerisation process.

Through the present invention, a polymer is obtained that is more optimised as regarding the mechanical and degradation properties compared to that of prior art. The material obtained through the present invention can be made stiffer. It is also rendered a lower speed of degradation. That is, degradation is slower. In other words, the strength of the material decreases slower than would be the case for conventional materials. How fast or slow the degradation goes, depends on the monomers chosen as staring materials.

According to one embodiment of the invention, R1 is derived from ethylene diamine, 1,3-diamino propane, 1,2-diamino propane, 1,4-diamino butane, 1,5-diamino pentane, or 1,6-diamino hexane.

According to one embodiment of the invention, R2 is derived from 4,4'diphenyl methane diisocyanate, naphthalene diisocyanate, or toluene diisocyanate.

According to one embodiment of the invention, R3 is derived from polycaprolactone diol, polydiethylene glycol adipate or poly (pentane diolpimelate).

The different monomers chosen for as R1 and R2 may be freely combined within the realms of the invention.

According to the invention, the linear block polymer may be spun into a fibre. The fibres may be produced by a wet spinning process described in " Gisselfält, K.; Flodin, P. Macromol. Symp. 1988, 130, 103-111 ".

Preferably, the fibres produced from the linear block polymer described above exhibits a specific strength of at least 0.1 N/Tex, more preferably above 0.2 N/Tex. The fibres exhibit high stiffness. Due to that fact, an implant made from the fibres may obtain a stiffness that makes the implant work very well as a replacement for the injured bodily tissue. For some implants, it is desirable that the elongation at break is not too high. Conventional polyurethane fibres of Spandex type, such as Lycra, often exhibit too high elongation at break. A fibre produced from the linear block polymer according to the invention preferably exhibits an elongation at break that is below 100 %.

The linear block polymer according to the invention may be used in different forms, depending on the use. Examples of suitable forms are fibres, foams and films. Other examples are porous films or porous polymeric material. Porous films are described in Swedish patent No. SE, C2, 514 064, which is hereby incorporated in its entirety. Further, porous film materials are described in Swedish patent application No. SE, A, 0004856-1, which is hereby incorporated in its entirety. SE, A, 0004856-1 describes a method for manufacturing an open porous polymeric material.

The invention further concerns an implant for the implantation into the human or animal body, which implant comprises a linear block polymer according to the invention.

### EXAMPLES

Example 1: 200 g (0.8 mol) 4,4'-diphenylmetandiisocyanate (MDI) was weighed in a flange flask. Nitrogen gas was applied and the MDI was slowly melted at 50°C. 202 g (0.38 mol) polycaprolacton diol (PCL) was weighed in a drop funnel and added during slow stirring, drop by drop, to the melted MDI. T=50-60°C.
24.6 g of the obtained prepolymer was dissolved under nitrogen gas in about 127.6 ml dimethyl formamid. 1.84 g (24.8 mmol) 1,3-diamino propane and 0.13 g (1.0 mmol) diamino butane was weighed in a beaker and added, together with 38.3 ml DMF, to the dissolved prepolymer during heavy stirring. A clear, viscous solution was obtained within seconds. Mₚₑₐₖ = 102 000 g/mol compared to PEO in DMF + 0.5 % LiCl.

Example 2: A prepolymer was manufactured according to Example 1, but with the modification that 1075.9 g (2.03 mol) PCL was mixed with 1035.2 g (4.14 mol) MDI. 20.34 g of the obtained prepolymer was dissolved under nitrogen gas in 84.3 ml dimethyl formamide (DMF). In the chain extension step 19.8 mmol 1,3-diamino propane, 0.51 mmol diamino butane and 21.1 ml DMF, was used. A clear, viscous solution was obtained within seconds. Mₚₑₐₖ = 106 000 g/mol compared to PEO in DMF + 0.5 % LiCl.

Example 3: 23.94 g prepolymer from Example 2 was dissolved in 101.7 ml DMF. In the chain extension step, 23.8 mmol 1,5-diamino pentane, 0.9 mmol diamino butane and 25.5 ml DMF was used. A clear, viscous solution was obtained within seconds. Mₚₑₐₖ = 106 000 g/mol compared to PEO in DMF + 0.5 % LiCl.

Example 4: A prepolymer was manufactured according to Example 1, with the modification that 1048.7 g (1.98 mol) PCL was mixed with 1041.2 g (4.06 mol) MDI. 18.96 g of the thus obtained prepolymer was dissolved under nitrogen gas in 68.2 ml dimethyl formamide (DMF). In the chain extension step 17.7 mmol 1,2-diamino propane, 3.1 mmol diamino butane and 29 ml of DMF, was used. A clear, viscous solution was obtained within seconds. Mₚₑₐₖ = 25 000 g/mol compared to PEO in DMF + 0.5 % LiCl.

Example 5: 27.18 g of the prepolymer obtained according to Example 1 was dissolved under nitrogen gas in 104 ml dimethyl formamide (DMF) and 1.23 g MDI was added. In the chain extension step 31.9 mmol 1,3-diamino propane, 1.3 mmol diamino butane and 44.6 ml DMF was used. A clear, viscous solution was obtained within seconds. Mₚₑₐₖ = 86 000 g/mol compared to PEO in DMF + 0.5 % LiCl.

### MEASUREMENTS OF MOLECULAR WEIGHT

The molecular weight of the polymers obtained from the above examples were measured by Size Exclusion Chromatography (SEC), with a Waters 2690 Separations Module provided with a Waters 996 Photodiode Array Detector and a Waters 2410 Refracive Index Detector. Two Styragel colons, HT6E and HT3, were run consecutively with a flow rate of 1 ml/minute of dimethyl formamide (DMF) comprising 0.005 g LiCl/l. The retention time was transformed into average molar mass (Mₚₑₐₖ), with the use of polyethylene oxide as a standard.

### FIBRE SPINNING

In short, the fibre spinning process comprises the steps of: the polymeric solution being extruded through a spinneret into a coagulation bath containing warm water; in a second water bath, the fibre is stretched; the fibre is rolled up on a spool, which is allowed to dry.

### THE MECHANICAL PROPERTIES OF THE FIBRE

The mechanical properties of the spun fibre were measured. The result is shown in the table below.

| Polymer. | Solvent spinning bath | Specific strength (N/Tex) | Stiffness x 10³ (N/mm) | Elongation at break (%) |
|---|---|---|---|---|
| Example 1 | DMF | 0.25 ± 0.015 | 50± 3 | 29± 4 |
| Example 2 | DMF | 0.28 ± 0.01 | 62± 4 | 40±3 |
| Example 3 | DMF+LiCl | 0.16 ± 0.015 | 56± 3 | 28±10 |

### DEGRADATION TESTS

Controlled degradation of the polymer in a pace that enables repair and/or ingrowth of bodily tissue are of great importance. The degradation circumstances for the linear block polymer of the invention was studied as buffered phosphate solution having a pH of 7.4. The temperature was kept at 77°C. During the degradation time, the mechanical properties of the polymer was measured.

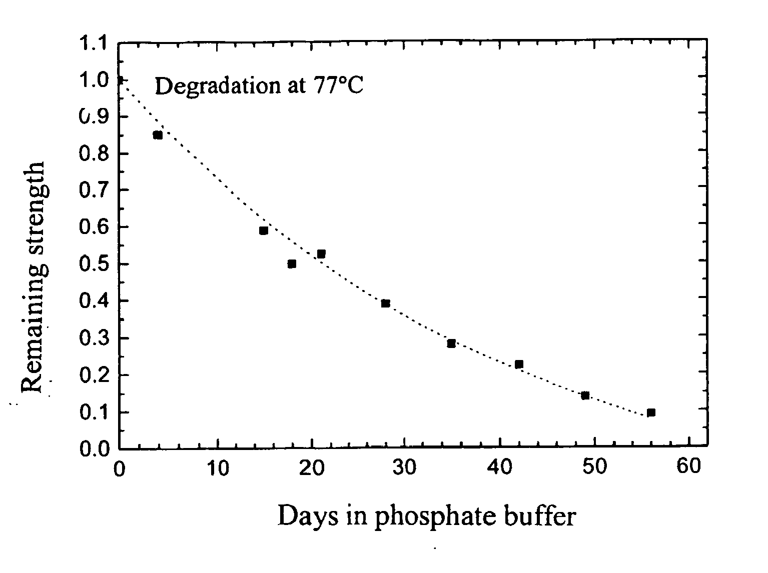

### FURTHER DESCRIPTION OF THE INVENTION

In the present study we describe the synthesis, wet spinning, mechanical testing and degradation of poly(urethane urea)s (PUUR) intended for clinical use in anterior cruciate ligament (ACL) reconstruction. The effects of soft segment chemical composition and molar mass, and the kind of diamine chain extender on the material properties were investigated. It was found that the fibres made of PUUR with polycaprolactone diol (PCL530) as soft segment and MDI/1,3-DAP as hard segment, (PCL530-3), have high tensile strength and high modulus, and when degraded keep their tensile strength for the time demanded for the application. In conclusion, from a chemical and mechanical point of view PUUR fibres of PCL530-3, ARTELON^{™}, are suitable for designing a degradable ACL device.

### Introduction

Ligament injuries in the knee joint are among the most common sporting injuries.^{1,2} Ruptures of the anterior cruciate ligament (ACL), the primary and most important stabiliser of the knee,³ are the most common serious ligament injuries. In the 1960s the first ACL reconstructions with synthetic materials were performed.⁴ The introduction of ligament prostheses generated much interest because it offered the benefit of quick recovery and rapid rehabilitation.⁵ While early results were promising, the long-term results were disappointing. A number of problems were reported, including irreversible elongation, rupture and formation of wear debris.

Materials used for these prosthetic devices or reinforcement ligament bands were e.g. poly(tetrafluoroethylene), poly(ethylene terephtalate),^{1,4,6-9} polypropylene, polyethylene, carbon fibres¹⁰ and polydioxanone.¹¹ The common properties of these materials are a too high elastic modulus compared to native ACL and permanent deformation after repeated loading due to non-elastic behaviour.

Materials with elastic behaviour and modulus above rubber level can be found among the multiblock copolymers. Poly(urethane urea)s (PUUR) are multiblock copolymers, which combine excellent mechanical properties with documented blood compatibility.^{12,13} These properties have favoured the use and development of PUUR as biomaterials, particularly as products for blood applications.¹⁴⁻¹⁷

PUURs are made of soft segments based on polyether or polyester and hard segments based on the reaction of diisocyanate and diamine chain extender. Due to the thermodynamic incompatibility between the two segments, PUURs undergo micro-phase separation resulting in the phase-separated heterogeneous structure that can be considered as hard segment domains dispersed in a soft segment matrix. The various physical properties of the material such as strength, modulus, and elasticity are closely correlated with the domain structure and the interaction between the segments inside the domain. By adjusting the chemical nature and respective amounts of reagents, it is possible to obtain a wide range of materials with different properties. Thus, materials may be tailored for various applications.

In designing a degradable device for anterior cruciate ligament (ACL) reconstruction, whether a true prosthesis or an augmentation device, many biological and mechanical criteria must be met. High initial strength is needed to prevent mechanical failure of the implant prior to tissue ingrowth.¹⁸ In addition, a moderate degradation rate is required to induce ingrowth of organized tissue.¹⁹ If degradation is too rapid, the host tissue may be exposed to stresses that are too great, resulting in failure. On the other hand, if the degradation is too slow, stress shielding may occur.¹⁹ Thus, a new material for ACL reconstruction should be 1) Compatible with surrounding tissues and allow cell ingrowth 2) Intended to be mechanically similar to native ACL 3) Degradable, but keeping at least 50% of its strength and stiffness for at least 9-12 months. A possible way to fulfil these requirements is to use a textile composition made of degradable PUUR fibres. Thus, the aim was to make PUUR fibres suitable for designing a degradable ACL device. Previously made PUUR fibres of the Spandex type, e.g. Lycra, are unsatisfactory for use as ligaments. In particular, their elastic modulus is too low and they are not degradable.

In this paper the synthesis, wet spinning, mechanical properties and degradation of a number of PUUR fibres are presented. The effects of soft segment chemical composition and content and the kind of diamine chain extender on the material properties are investigated.

### Experimental section

**Materials.** Polycaprolactone diols (PCL) (M̅ₙ= 530 g/mol) and (M̅ₙ= 1250, 2000 g/mol) were obtained from Solvay and Aldrich, respectively. Adipic acid, di(ethylene glycol), di-n- butylamine, ethylene diamine (EDA), 1,2-diaminopropane (1,2-DAP), 1,3-diaminopropane (1,3-DAP), 1,4-diaminobutane (1,4-DAB), 1,5-diaminopentane (1,5-DAPe), 1,6-diaminohexane (1,6-DAH) and lithium chloride (LiCl) were purchased from Fluka. 4, 4'-diphenylmethane diisocyanate (MDI) was provided by Bayer AB. N, N-dimethylformamide (DMF) 99.8% and toluene 99.8% were obtained from Labscan.

**Polyester synthesis.** Hydroxytelechelic polyesters were synthesized from adipic acid and di(ethylene glycol) with acid catalyst until the acid number was < 2 as determined by titration of aliquots with 0.1 molar KOH in ethanol. The removal of water to drive the reaction at a reasonable rate was achieved by azeotropic distillation with toluene. Three products with hydroxyl numbers of 56 (M̅ₙ =2000 g/mol), 112 (M̅ₙ =1000 g/mol) and 223 (M̅ₙ =500 g/mol), respectively, as determined according to ASTM D 4274-94, were used in the present study.

**Polymerization.** PUURs ²⁰ were synthesized by a two-step method described earlier.²¹ In the first step a prepolymer was formed. The polyester diol was added slowly to 4, 4'-diphenylmethane diisocyanate (MDI) (NCO: OH=2.05:1) in bulk at 50°C in a dry N₂ atmosphere. The isocyanate content was determined by reacting the prepolymer with an excess of di-n-butylamine in toluene. After the reaction was complete, the excess di-n-butylamine was determined by back titration with standard hydrochloric acid.

In the second step a dilute solution of diamine chain extender and monoamine chain stopper in DMF was added rapidly to a solution of prepolymer in DMF (20 wt.-%) under intense stirring at 20°C. The molar ratio NCO:NH₂ was 1:1 with 2% monoamine. The final polymer content was 18 wt.-%. The chemical compositions of the various PUUR can be seen in Table 1.

**Fibre spinning.** Fibres were prepared by a wet spinning process²¹ (equipment from Bradford University Research Limited, Bradford, England). The polymer solution was metered through a spinneret (120 holes, ∅ 80 µm) submersed in a coagulating bath containing water. In a second water bath the fibre bundle was drawn after which the multifilament fibre was taken up on a spool. The temperature in the water baths was varied from 20 to 80°C to get as high draw ratio as possible. The spools with fibres were rinsed in running tap water overnight and dried at room temperature. For each batch of fibres linear density, tensile strength, stiffness and elongation at break were determined.

**Band production.** The wet spun multifilament fibres were by doubling and slight twisting converted to a coarse yam, which was used as warp threads. The bands were woven on a narrow fabric needle loom (type FX2/65, Mageba Textilmaschinen Vertriebs GMBH, Germany) with low weft tension in plain weave to utilise as much as possible of the yarn strength combined with good stability.

**Density measurements.** The density of the fibres was measured with a Micrometrics Multivolume Pycnometer 1305.

**Porosity measurements.** Pore sizes and pore size distributions of the woven bands were measured by mercury porosimetry, Micromeretics AutoPore III 9410.

**Polymer degradation.** Samples of fibres and bands were placed in vials in a great surplus of 0.06 M phosphate buffer solution pH 7.4 (Na₂HPO₄ and KH₂PO₄.²² The sealed vials were placed in thermostat ovens at 37°C and 77°C. At intervals the vials were opened and aliquots of material were taken out. Changes in molar mass and loss of tensile strength were investigated.

**Size Exclusion Chromatography (SEC).** Size Exclusion Chromatography (SEC) was conducted with a Waters 2690 Separations Module equipped with a Waters 996 Photodiode Array Detector and a Waters 2410 Refractive Index Detector. Two Styragel columns, HT6E and HT3, were operated in series at a flow rate of 1 ml/min in DMF containing 0.5%(w/v) LiCl to prevent aggregation. The retention times were converted to apparent molar masses using poly(ethylene oxide) standards.

**Linear density measurements.** The linear density of the fibres was determined by weighing of a known length of fibre, typical 100 m, and is presented in tex. The tex unit is defined as g/1000 m.

**Mechanical testing.** After equilibration with water at 20°C for 30 minutes, the multifilament fibres and the woven bands were tested in the wet state in a tensile tester (UT 350/5 LS Universal Testing machine SDL International Ltd. Stockport). The constant rate of extension was 900 mm/minute and the sample lengths were 100 mm for fibres and 30 mm for the woven bands.

**Differential Scanning Calorimetry (DSC).** Thermal analysis was carried out on a Perkin-Elmer Pyris1. The heating rate was 10°C /min over a temperature range of-100 to 150°C. The sample was cooled to -100°C and then a second run was performed. Glass transition temperatures were determined from the second scan.

### Results and Discussion

**Polymerization.** In a first study, PUURs were synthesized using poly(di(ethylene glycol) adipate) (PDEA) or polycaprolactone diol (PCL), with different molar masses as soft segments, MDI and EDA as chain extender (Table 1). The length of the soft segment was altered by changing the molar mass of the polyesterdiol while the length of the hard block was unchanged. However, there was a distribution of hard block lengths as a consequence of the stoichiometric ratio in the prepolymerization step.²³ As the soft segment was shortened from 2000 g/mol to 500 g/mol the hard block content increased from 23 % by weight to 55%. An immediate effect was that the solubility in DMF decreased with increasing hard block content, resulting in turbidity and gelation a few minutes after chain extension.

Another series of PUURs was prepared using PCL530 as soft segment, MDI and six different aliphatic diamines as chain extenders (Table 1).

**Table 1. Composition and solubility of PUURs.**

| Sample code | Chain extender | Soft segment M̅ₙ | Hard block content (%) | DMF solution (18%, 22°C) | Mₚₑₐₖ x 10^{-3 *c*} |
|---|---|---|---|---|---|
| PDEA2000-2 | EDA | 2000 | 23.0 | Opaque^{a} | 86 |
| PDEA1000-2 | EDA | 1000 | 37.7 | Opaque | 86 |
| PDEA500-2 | EDA | 500 | 55.9 | Opaque | 115 |
| PCL2000-2 | EDA | 2000 | 23.0 | Opaque | 105 |
| PCL1250-2 | EDA | 1250 | 33.0 | Opaque | 121 |
| PCL530-2 | EDA | 530 | 51.4 | Opaque | 125 |
| PCL530-2Me | 1,2-DAP | 530 | 52.0 | Clear^{b} | 106 |
| PCL530-3 | 1,3-DAP | 530 | 52.0 | Clear | 125 |
| PCL530-4 | 1,4-DAB | 530 | 53.0 | Opaque | 123 |
| PCL530-5 | 1,5-DAPe | 530 | 53.6 | Opaque | 106 |
| PCL530- 6 | 1,6-DAH | 530 | 54.2 | Opaque | 106 |

| | | | | | |
|---|---|---|---|---|---|
| a) Opaque: hazy, poorly solubilized in the above condition. b) Clear: transparent, absolutely solubilized in the above condition. c) Poly(ethylene oxide) equivalent Mₚₑₐₖ. | | | | | |

The different chain extender structures affected the solubility of the polymer in DMF. These PUURs have almost the same hard/soft ratio and showed solubility in the order 1,2-DAP>1,3-DAP>1,5-DAPe>1,6-DAH>1,4-DAB>EDA. In the reactions all diamines but 1,2- and 1,3-DAP gave rise to turbid solutions 5-20 minutes after chain extension. After still some time brittle gels were formed. 1,3-DAP formed clear polymer solutions, but they were turbid and gelled after a few days. PCL530-2Me solutions remained clear for at least one year. This is the most apparent difference between 1,2-DAP and the other five chain extenders and is explained in terms of less efficient hydrogen bonding due to steric effects from the pendant methyl group. Similar behaviour has been seen for PUUR systems chain extended with aromatic diamines with substituents that increased the steric effects.²⁴

The lower solubilities of the other PUURs probably depend on the influence of the urea structure on the association behaviour. PUUR solutions with even number of methylene groups in the chain extender got turbid very soon while odd numbered remained clear for longer times. Similar results were found by Joel et al.,²⁵ who studied steric odd-even effects of various urea structures of PUUR on solution properties in DMF. They found that the viscosity of solutions with odd number of methylene groups was independent of time, while the even numbered ones showed turbidity and a drastic increase in viscosity with time followed by gelation. The turbidity was explained by formation of a higher concentration of physical crosslinks caused by hydrogen bonding within the hard block domains.²⁶ The solution process becomes restricted if the hard segment domains are perfectly arranged and form a physically crosslinked network. Thus, the good solubility of PCL530-3 may be explained by a lower degree of hydrogen bonding compared to the other chain extenders.

A requirement for spinnability is that the polymer is soluble. The solvent, DMF, should prevent gelation due to hard segment interaction before spinning, but the solubility of PUUR in DMF is poor. By adding LiCl (0.07 g LiCl /g polymer solution) to the polymer solutions turbidity could be removed and gelation could be prevented.²⁵ The increased solubility is based on the destruction of the hydrogen bonds between chains and on a simultaneous blocking of the acceptor positions owing to the favoured complex formation between Li and carbonyl oxygen.²⁷

**Fibre spinning.** The fibres are formed in a wet spinning process. In the first step precipitation occurs and the solvent diffuses out of the extrudate into the bath, and non-solvent diffuses from the bath into the extrudate. The rate of the coagulation has a profound effect on the yarn properties. Important process variables are for example concentration and temperature of the spinning solution, composition and temperature of the coagulation bath.

The temperature of the spinning solutions was kept within 20-25°C and the polymer concentration was 18 wt.-%. No correlation between polymer content and tensile properties could be seen. However, a spinning solution viscosity of more than 1 Pas was needed to be able to get a stable spinning process.

The temperature of the coagulation bath was found to be of great importance. The rate of PUUR coagulation occurring when the polymer solution was extruded into the water depends on the coagulation temperature and influences both the morphology of the undrawn fibre and the ultimate fibre properties. The suitable spin bath temperature for PDEA based PUURs was about 20°C (Table 2). At higher temperatures the polymer got stuck in the spinneret. In contrast the PCL based PUURs seemed to be easier to spin the higher the temperature (Table 2). This difference between the two polyesters can be due to their difference in hydrophilicity ²⁸.

In the second water bath the fibre bundle is drawn to get molecular chain orientation and thereby improve the mechanical properties. The higher the draw ratio, the lower the elongation and the stiffer and stronger the fibre.

The effect of draw ratio on tensile properties for PCL530-3 is seen in Figure 1. 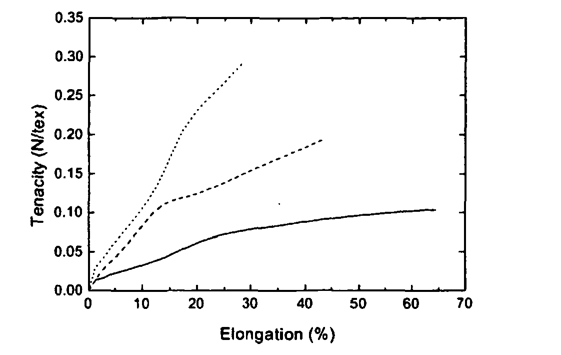 **Figure 1.** Tensile test diagrams for PUUR fibre of PCL530-3 showing changes produced by increasing orientation and improving structure. Draw ratio □ 3.5, □ 3.8 and □ 5.4.

**Table 2. Spin parameters for different PUURs**

| Sample code | Spin solvent | T_{draw} optimal^{a} (°C) | Draw ratio_{20°C}/Draw ratio_{Tdraw optimal} | Draw ratio |
|---|---|---|---|---|
| PDEA2000-2 | DMF+LiCl | 20 | - | 5 |
| PDEA 1000-2 | DMF+LiCl | 20 | - | 5 |
| PDEA500-2 | DMF+LiCl | 20 | - | 4.5 |
| PCL2000-2 | DMF+LiCl | 60 | 0.58 | 6 |
| PCL1250-2 | DMF+LiCl | 60 | 0.57 | 6 |
| PCL530-2 | DMF+LiCl | 60 | 0.72 | 5 |
| PCL530-2Me | DMF | 60 | 0.67 | 9 |
| PCL530-3 | DMF | 60 | 0.72 | 5.4 |
| PCL530-3 | DMF+LiCl | 80 | 0.44 | 5.4 |
| PCL530-4 | DMF+LiCl | 80 | 0.70 | 5.4 |
| PCL530-5 | DMF+LiCl | 80 | 0.46 | 6.4 |
| PCL530- 6 | DMF+LiCl | 80 | 0.54 | 7.4 |

| | | | | |
|---|---|---|---|---|
| a) T_{draw optimal}= the temperature at which the highest draw ratio is achieved | | | | |

The draw ratio of the fibres is dependent on the temperature not only in the coagulation bath but also in the stretching bath. It was found that the best processability and draw ratio were achieved when the baths had the same temperature. The spinning conditions are shown in Table 2. Three different groups are identified. The first group contains PDEA based PUURs, described earlier, which have best processability and draw ratio at 20°C. The second group contains PCL based PUURs chain extended with EDA spun from DMF+LiCl, and PCL530-2 Me and PCL 530-3 spun from DMF. These obtain their highest draw ratio at 60°C and their drawability is directly proportional to the temperature. In the third group the highest draw ratio is achieved at 80°C, maybe it is possible to increase their draw ratio further if the temperature is raised further. This group contains PCL- based PUUR with chain extenders with more than two methylene groups and are spun from DMF+LiCl. Even though the highest draw ratio was achieved at the same temperature within each group, the temperature dependence of draw ratio of the fibres differed (Table 2 and Figure 2 a, b). 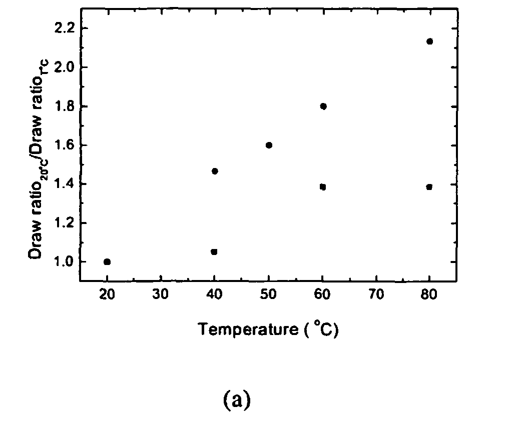 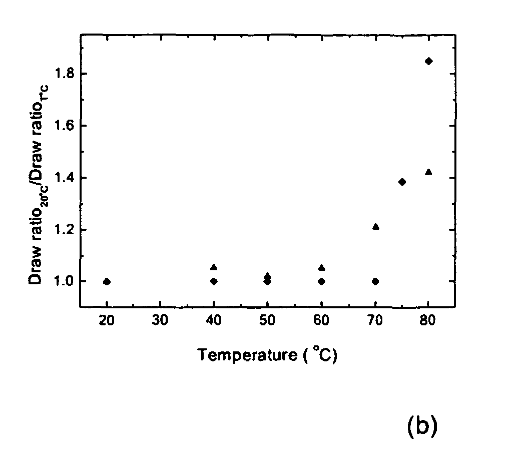 **Figure 2.** Draw ratio_{20°C} /Draw ratio_{T°C}; a)(!) PCL530-3 and (,) PCL530-5; b) (7) PCL530-4 and (A) PCL530-6.

The increase in draw ratio of PCL530-5 was almost proportional to the temperature, while the increase in draw ratio of PCL530-3 showed weak temperature dependence between 20°C and 50°C. Above that interval the drawability was directly proportional to the temperature. The drawability of PCL530-4 and PCL530-6 was constant at temperatures below 60°C and 70°C, respectively. At these temperatures strong temperature dependence in drawability appeared. Additional investigations are needed to explain these differences.

**Band production.** The appropriate force at break of the finished and sterilised band should be 1200 N. Based on practical experiences the theoretical breaking force therefore was chosen to 1600 N in order to calculate the resulting cross section of the band as well as the number of fibres needed. Furthermore the diameter of the band was not allowed to exceed 5±1 mm. In the finished band three circular yarns are placed in a triangular form. Assuming hexagonal close packing of the fibres in the yarn,²⁹ the yarn radius can be calculated. From the calculations it is given that the tenacity of the fibres should be at least 0.2 N/tex to meet the criteria of strength and size.

**Porosity measurements.** In medical applications the pore sizes and their distributions are of great importance for promoting cell ingrowth. The multifilament fibres made from wet spinning have a high void content. Furthermore, when fibres are processed into woven structures, varying degrees of porosity can be provided. The pore sizes and pore size distribution of two woven bands made of 1500 multifilament PCL530-3 fibres are presented in Table 3. The smallest pores, <8 µm, is probably between the filaments in the multifilament fibre while pores between 8 µm and 600 is the space between the fibres in the warp and weft (Figure 3). Almost half of pores (49%) are 21-100 µm, sizes that may be suitable for fibrous connective tissue ingrowth.³⁰ About 20% of the pores are 100-400 µm, pore sizes, which have been shown to be suitable for osteoblast ingrowth in hard tissue applications.³¹

**Table 3. Pore sizes and pore size distributions of PUUR bands.**

| Interval (µm) | Pores (%) |
|---|---|
| 401-600 | 6.8 |
| 201-400 | 15.6 |
| 101-200 | 8.0 |
| 51-100 | 20.7 |
| 21-50 | 28.3 |
| 8-20 | 10.7 |
| 1.0-7.9 | 4.5 |
| <1 | 5.4 |

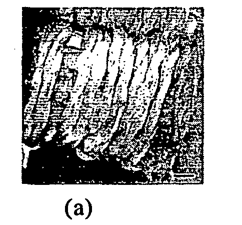

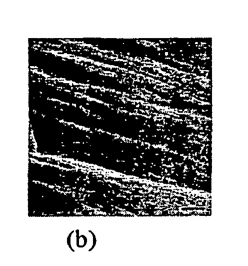

**Figure 3.** SEM micrographs of the surface of a) wet spun and woven PUUR fibre bar=50 µm; b) wet spun PUUR fibre bar=5 µm.

**Mechanical testing.** The tensile properties of the fibres are shown in Table 4. In the first series the length and composition of the esterdiol was varied and the hard block was formed from MDI and EDA. The effects upon shortening the soft segment were seen in increased stiffness and decreased elongation of the fibres. The largest effect is seen when comparing polymers made from soft segments of molar masses of ∼1000 g/mol and ∼500 g/mol. As a consequence of the shortening of the soft segment the hard/ soft ratio increases. The hard blocks, which are extensively hydrogen bonded, mainly affect the stiffness and serve as both cross-links and filler particles in the soft segment matrix. It is known that the strength and modulus of polyurethane copolymers are directly related to the amount and stability of the hard segment domains.³² Wang and Cooper³² studied the effect of the hard block content and block length on the morphology and properties of PUURs systematically. They found that the mechanical properties depended primarily on the hard block content and the strong hard-domain cohesion due to interurea hydrogen bonding that resulted in semicrystalline behaviour.

The effects on the mechanical properties of the PCL 530 based PUUR fibres chain extended with six different aliphatic diamines were investigated (Table 4). The hard segment content is almost constant, but their structures differ. The strongest fibres were obtained from PCL530-2Me and PCL530-3, which are supposed to be less efficiently hydrogen bonded in solution compared to the other PUURs. PCL530-2Me and PCL530-3 formed clear solutions and could be spun without addition of LiCl. As LiCl was added to a PCL530-3 solution the tenacity of the fibre produced thereof decreased more than 40%. Nevertheless, the tenacity of PCL530-3 fibre from DMF+LiCl was still among the highest. Upon a comparison of fibres spun from DMF-LiCl solutions PCL530-3 and PCL530-5 are the strongest. Thus, chain extenders with an odd number of methylene groups tend to form stronger fibres than those with even numbers. From the design of the ACL reconstruction band it was given that the fibre should have a tenacity of at least 0.2 N/tex. Only two of the tested fibres, made from PCL530-3 and PCL530-2Me, met this requirement.

**Table 4. Mechanical properties of different PUUR fibres.**

| Sample code | Spin solvent | Tenacity (N/tex)^{a} | Stiffness x 10³ (N/mm) | Elongation at break (% |
|---|---|---|---|---|
| PDEA2000-2 | DMF | 0.06 ± 0.004 | 6± 0.5 | 130±13 |
| PDEA1000-2 | DMF | 0.06 ±0.004 | 6± 0.4 | 120±10 |
| PDEA500-2 | DMF+LiCl | 0.08 ±0.005 | 17± 1 | 50± 5 |
| PCL2000-2 | DMF+LiCl | 0.13 ± 0.007 | 6± 0.3 | 77±12 |
| PCL1250-2 | DMF+LiCl | 0.11 ± 0.005 | 6± 0.4 | 74±14 |
| PCL530-2 | DMF+LiCl | 0.10 ± 0.01 | 45± 2 | 32± 8 |
| PCL530-2Me | DMF | 0.25 ± 0.015 | 50± 3 | 29± 4 |
| PCL530-3 | DMF | 0.28 ± 0.01 | 62± 4 | 40±3 |
| PCL530-3 | DMF+LiCl | 0.16 ± 0.008 | 50± 3 | 34± 2 |
| PCL530-4 | DMF+LiCl | 0.10 ± 0.01 | 60± 3 | 25±3 |
| PCL530-5 | DMF+LiCl | 0,16 ± 0.015 | 56± 3 | 28±10 |
| PCL530- 6 | DMF+LiCl | 0.11 ± 0.01 | 70± 3 | 16±5 |

| | | | | |
|---|---|---|---|---|
| ^{a)} Density of fibres=1.23 g/cm³ | | | | |

**Bands.** Typical tensile test diagram of a band of PCL 530-3 fibres is given in Figure 4. The shape of load-elongation curve of the band is similar to that of the fibre, but the elongation is higher. This is expected as the fibres are slightly twisted to get a coarse yarn before weaving. 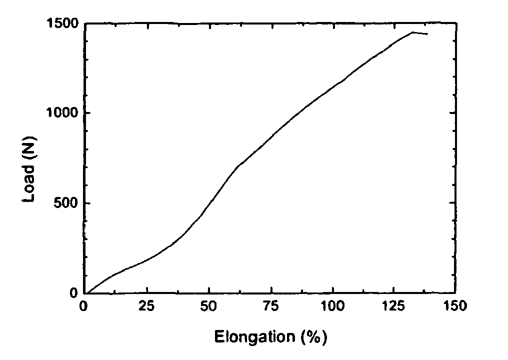 **Figure 4.** Typical tensile test diagrams for a PUUR band of PCL530-3 fibres.

**Differential Scanning Calorimetry.** The glass transition temperatures of the soft blocks for the different PUURs are presented in Table 5. As the molar mass of the soft segments decreased the T_{g} increased from -30.7 to -5.5°C and from -48.3 to -11.3°C for PDEA and PCL respectively. The T_{g} was harder to detect the shorter the soft segment. The effect of soft segment length on its T_{g} is related to the limitation of the soft segment mobility imposed by the attached hard segment. Beside the molar mass of the soft segment, the chain extender affected the T_{g} so some extent. PCL530-2Me and PCL530-3 showed the highest T_{g}. For chain extenders with three or more methylene groups there was a movement towards higher T_{g} the longer the diamine. All PUUR fibres but PCL530-2Me and PCL530-3 were spun from DMF+LiCl. PCL530-3 spun from DMF+LiCl showed no change in T_{g} compared to PCL530-3 spun from DMF. The phase mixing of soft and hard segments makes thermal molecular motion in soft segment phase restricted. Therefore, the shifts of T_{g} to higher temperatures are attributed to the interaction between hard and soft segments.

**Table 5. DSC data**

| Sample code | Soft segment T_{g} (°C) |
|---|---|
| PDEA2000-2 | -30.7 |
| PDEA1000-2 | -25.7 |
| PDEA500-2 | -5.5 |
| PCL2000-2 | -48.2 |
| PCL1250-2 | -39.1 |
| PCL530-2 | -11.3 |
| PCL530-2Me | -9.2 |
| PCL530-3 | -8.4 |
| PCL530-3 (LiCl) | -8.3 |
| PCL530-4 | -10.2 |
| PCL530-5 | -11.2 |
| PCL530- 6 | -13.7 |

**Degradation studies.** Among the various bonds present in PUUR, the most susceptible ones are the ester bonds of the soft segments which upon hydrolysis yield carboxylate and hydroxyl groups. The acid produced can catalyse the ester hydrolysis so that an autocatalytic reaction becomes prevalent. Furthermore, the intended use for the material is in the knee that has a neutral pH.³³ For that reason all degradation studies were carried out in a great surplus of buffered solutions at pH 7.4. Both changes in molar mass and tensile strength of optimal drawn fibres and bands thereof were studied. The data for fibres and bands obey the same dependence on change in molar mass and tensile strength. Thus, the difference in fibre packing and sample thickness do not seem to have any effect on degradation rate.

The loss in tensile strength with hydrolysis time at 77°C is shown in Figure 5. 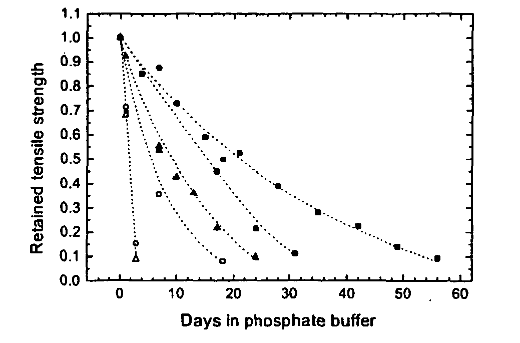 **Figure 5.** Tensile strength retention as a function of hydrolysis time of different PUURs at 77°C (!) PCL530-2 and PCL530-3, (,) PCL1250-2, (7) PCL2000-2, (V) PDEA500-2, (-) PDEA1000-2, (8) PDEA2000-2.

For both PDEA- and PCL-based PUUR the polymer with longer soft segments degraded faster. Thus, PDEA2000 and PCL2000 display lower hydrolytically stability than PCL530 and PDEA500, respectively. The reason is a higher fraction of soft segments and, consequently, of ester groups exposed to hydrolysis.

The chemical composition of the ester affects the degradation rate of the different PUURs. PUURs with soft segments made of PDEA degrade faster than those based on PCL. The superior resistance to hydrolysis is ascribed to the hydrophobicity of PCL. It has been shown that the introduction of hydrophilic poly(oxyethylene) blocks in PCL-POE-PCL triblock copolymers did increase the hydrophilicity and degradation rate compared with the homopolyester PCL²⁸. PDEA 500 contains about three diethylene glycols whereas the PCL diols initiated with diethylene glycol contain one.

The initial molar mass of the different PUURs varied to a small extent (Table 1). The rate of the decrease in tensile strength was not affected, but the time to complete degradation became somewhat shorter the lower the initial molar mass. The molar mass decreases after an induction period of about 10 days for PCL530-2 and-3 and about 3 days for PCL1250-2 (Figure 6). No induction period was seen for the other samples. During the induction period a decrease in SEC retention time was seen. The phenomenon has been occasionally reported by some researchers using both *in vitro* and *in vivo* systems,³⁴⁻³⁶ but no unanimous conclusions on the reasons for the increase were drawn. We have interpreted the decrease in retention time, as aggregation of the polymer due to a physical process since no unreacted isocyanate groups is present in the polymers. Furthermore, in spite of the increase in molecular size the tensile strength decreased already after 2 days in phosphate buffer (Figure 5). 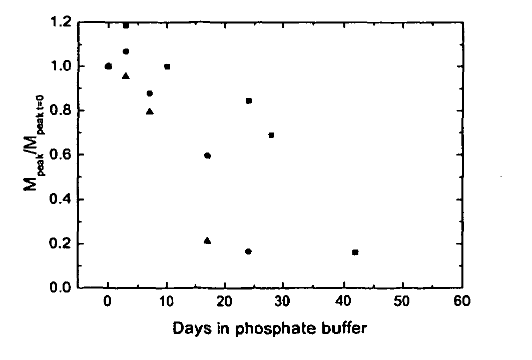 **Figure 6.** Molar mass as a function of hydrolysis time of different PCL based PUURs at 77°C (!) PCL530-3, (,) PCL1250-2, (7) PCL2000-2.

One of the requirements of the material was that at least 50 % of the tensile strength should be kept for at least 9-12 months. For use at body temperature it is interesting to estimate and investigate the conformity between the degradation rate at 37°C and 77°C.

Since fibres of PCL530-3 seem to have the most promising properties from many aspects further degradation studies at 37°C were made these fibres. The change in molar mass at 37°C follows the same pattern as at 77°C. After about 40 days a decrease in SEC retention time for the polymer is seen, which is similar to the results after 2-3 days at 77°C. After that the retention time is constant for a long period of time. This means that the size of the formed aggregates is to some extent independent of the molar mass of the molecules that take part in the aggregate formation. It is obvious that the degradation proceeds since after 500 days the SEC retention time has increased accompanied by a the loss in tensile strength of 5-10%. Thus, the degradation rate at 37°C seems to be about 1/20 lower than of the degradation rate at 77°C.

In addition to loss of tensile strength and molar mass, the mass change of the PCL530-3 fibres has been studied. First the mass of the fibres increased about 8-10% due to water adsorption. After 52 days at 77°C there were signs of mass loss and after another ten days the mass loss is obvious (-30%). At that time the fibres were very brittle at and further measurements were hard to perform. In accordance with the low degradation rate at 37°C it is not expected to see any decrease in the mass before 800-850 days of degradation.

Regarding the biocompatibility of the PCL530-3 both safety studies, mutagenicity and delayed contact hypersensitivity, and implantation studies have been performed and reported.³⁷ When PUUR bands made of PCL530-3 fibres were used as ACL prosthesis in rabbits and minipigs, bone formation was seen in the drilled tunnels and surrounding the PUUR fibres. Also, irrespective of observation time, connective tissue in growth was observed between and in close contact with the PUUR fibres in both species. At observation times exceeding 6 months, the connective tissue had an orientation of the collagen fibres and fibroblasts in parallel with the PUUR fibers.³⁷ From clinical trials using the PUUR band as an ACL augmentation, biopsies were obtained from 5 patients at times between 30 to 40 months. As in the animals a high percentage of connective tissue ingrowth was found in close contact with the material and the presence of collagen type I and blood vessels was confirmed using immunohistochemical methods. No indications of obvious inflammatory reaction or foreign body response were detected.

**Conclusions.** We have demonstrated that the chemical composition of PUUR can be tailored to get fibres with strength, stiffness and degradation rate, which fulfil the desired properties of a material for ACL reconstruction.

Fibres made of PUUR based on PCL 530 have superior strength and stiffness compared to other polyesterdiols used in the study and keep at least 50 per cent of its original tensile strength more than nine months at body temperature. Furthermore, chain extension of PCL530:MDI with 1,3-DAP produces a polymer solution from which strong fibres can be spun without additives. A porous band with appropriate strength and size can be woven of the fibres.

In conclusion, from a chemical and mechanical point of view fibres of PCL530-3, ARTELON^{™}, are suitable for designing a degradable ACL device. Human clinical trials with ACL reconstruction using the PUUR band are in progress.

**Acknowledgement.** The Knowledge Foundation via the Material Research School at Chalmers University of Technology is gratefully acknowledged for financial support.

### References

(1) Fu, F.; Bennett, C. H.; Lattermann, C.; Ma, C. *Am. J. Sports. Med.* **1999**, *27*, 821-830.
(2) Feagin, J. A. J.; Lambert, K. L.; Cunningham, R. R.; Anderson, L. M.; Riegel, J.; King, P. H.; VanGenderen, L. *Clin. Orthop.* **1987,** *216*, 13-18.
(3) Feagin, J. A. J. *The Crucial Ligaments.;* Churchill Livingstone Inc.: New York, 1988.
(4) von Mironova, S. S. *Zentralbl. Chir.* **1978,** *103,* 432-434.
(5) Mody, B.; Howard, L.; Harding, M.; Parmar, H.; Learmonth, D. *J. Bone Joint Surg. [Br]* **1993**, *75*, 18-21.
(6) Rading, J.; Peterson, L. *Am. J. Sports. Med.* **1995**, *23*, 316-319.
(7) Wredmark, T.; Engström, B. *Knee Surg., Sports Traumatol. Arthrosc.* **1993**, *1*, 71-75.
(8) Engström, B.; Wredmark, T.; Westblad, P. *Clin. Orthop.* **1993,** *295*, 190-197.
(9) Denti, M.; Bigoni, M.; Dodaro, G.; Monteleone, M.; Arisio, A. *Knee Surg., Sports Traumatol. Arthrosc.* **1995**, *3*, 75-77.
(10) Rushton, N.; Dandy, D. J.; Naylor, C. P. E. *J. Bone Joint Surg. [Br]* **1983**, *65*, 308-309.
(11) Puddu, G.; Cipolla, M.; Cerullo, G.; Franco, V.; Gianni, E. *Clin. Orthop.* **1993**, *12*, 13-24.
(12) Boretos, J. W.; Pierce, W. S. *Science* **1967**, *158*, 1941.
(13) Lyman, D. J.; Knutson, K.; McNeil, B.; Shibatani, T. *Trans. Am. Soc. Artif. Intern. Organs* **1975**, *21*, 49-54.
(14) Lyman, D.; Fazzio, F.; Voorhes, H.; Robinson, G.; Albo, D. J. *J. Biomed. Mater. Res.* **1978**, *12*, 337-345.
(15) Farrar, D.; Litwak, P.; Lawson, J.; Ward, R.; White, K.; Robinson AJ; Rodvien R; Hill, J. *J. Thorac. Cardiovasc. Surg.* **1988,** *95,* 191-200.
(16) Lawson, J. H.; Olsen, D.; Hershgold, E.; Kolff, J.; Hadfield, K.; Kolff, W. J. *Trans. Am. Soc. Artif. Intern. Organs* **1975,** *21,* 368-373.
(17) Durst, S.; Leslie, J.; Moore, R.; Amplatz, K. *Radiology* **1974,** *113*, 599-600.
(18) Dunn, M. G.; Avasarala, P. N.; Zawadsky, J. P. *J. Biomed Mater. Res.* **1993**, *27*, 1545-1552.
(19) Silvaggio, V. J.; Fu, F. H. In *Articular Cartilage and Knee Joint Function: Basic Science and Arthroscopy;* Ewing, J. W., Ed.; Raven Press, Ltd: New York, 1990; pp 273-299.
(20) USA patent no. 6210441, 2001-04-03
(21) Gisselfält, K.; Flodin, P. *Macromol. Symp.* **1998,** *130,* 103-111.
(22) *International Organisation for Standardisation. International standard ISO 13781:1997(E),* 1997.
(23) Petrovic, Z. S.; Ferguson, J. *Prog. Polym. Sci.* **1991**, *16*, 695-836.
(24) Lee, H. K.; Ko, S. W. *J. Appl. Polym. Sci.* **1993**, *50*, 1269-1280.
(25) Joel, D.; Pohl, G.; Hiller, W. G. *Angew. Makromol. Chem.* **1993,** *208*, 107-116.
(26) Bonart, R.; Morbitzer, L.; Müller, E. H. *J. Polym. Sci., Polymer Phys.* **1974,** *B9,* 447-461.
(27) Spevacek, J.; Hiller, W. G.; Hettrich, W.; Joel, D. *Eur. Polym. J.* **1989,** *25***,** 1239-1243.
(28) Li, S.; Garreau, H.; Vert, M.; Pertrova, T.; Manlova, N.; Rashkov, I. *J*. *Appl. Polym. Sci.* **1998,** *68,* 989-998.
(29) Hearle, J. W. S.; Grosberg, P.; Backer, S. *Structural Mechanics of Fibers, Yarns, and Fabrics;* Wiely-Interscience: New York, 1969; Vol. 1.
(30) Pollock, E.; Andrews, E.; Lentz, D.; Skeikh, K. *Trans. Am. Soc. Artif. Intern. Organs* **1981**, *27*, 405-409.
(31) Schiephake, H.; Neukam, F. W.; Klosa, D. *Int. J. Oral. Maxillofac. Surg.* **1991,** *20*, 53-58.
(32) Wang, C. B.; Cooper, S. L. *Macromolecules* **1983**, *16*, 775-786.
(33) Cummings, N. A.; Nordby, G. L. *Arthritis. Rheum.* **1966**, *9*, 47-56.
(34) Xi, T.; Sato, M.; Nakamura, A.; Kawasaki, Y.; Umemura, M.; Tsuda, M.; Kurokawa, Y. J. *Biomed. Mater. Res.* **1994**, *28*, 483-490.
(35) Wu, L.; Weisberg, D.; Felder, G.; Snyder, A.; Rosenberg, *G. J. Biomed. Mater. Res.* **1999**, *44*, 371-380.
(36) Wu, L.; Li, D.; You, B.; Qian, F. *J. Appl. Polym. Sci.* **2001,** *80*, 252-260.
(37) Liljensten, E.; Gisselfält, K.; Nilsson, A.; Lindahl, A.; Edberg, B.; Bertilsson, H.; Flodin, P.; Peterson, L. *J. Mater. Sci.-Mater. M.* **2002,** *13*, 351-359.

## Claims

1. Linear block polymer according to Formula (1) 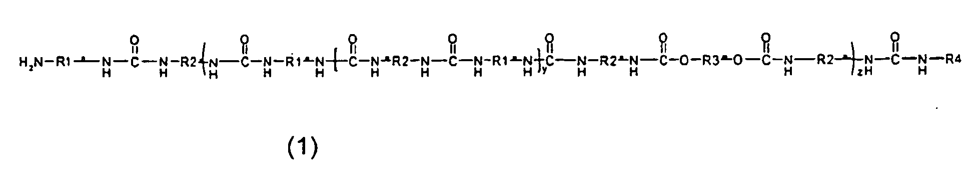 Wherein
R1 is derived from a diamine, e.g. ethylene diamine, 1,2-diamino propane or 1,3-diamino propane;
R2 is derived from an aromatic diisocyanate;
R3 is derived from an esterdiol;
R4 is derived from dibutyl amine or ethanolamine;
Where 0 < y < 4 and z > 8,
**characterized in that**,
the monomers from which R2 and R3 are derived from are added in such amounts that the molar ratio between R2 and R3 is larger than 2:1.

2. Linear block polymer according to claim 1, wherein R1 is derived from ethylene diamine, 1,3-diamino propane, 1,2-diamino propane, 1,4-diamino butane, 1,5-diamino pentane, or 1,6-diamino hexane.

3. Linear block polymer according to claim 1 or 2, wherein R3 is derived from polycaprolactone diol, polydiethylene glycol adipate or poly(pentane diolpimelate).

4. Linear block polymer according to any of the preceding claims, wherein R2 is derived from 4,4'diphenyl methane diisocyanate, naphthalene diisocyanate, or toluene diisocyanate.

5. Fibre manufactured from a linear block polymer according to any of the preceding claims.

6. Fibre according to claim 5, which fibre exhibits a toughness of at least 0.1 N/Tex.

7. Fibre according to claim 6, which fibre exhibits a toughness above 0.2 N/Tex.

8. Fibre according to any of claims 5-7 which fibre exhibits an elongation at break that is below 100 %.

9. Fibre according to any of claims 5-7 which fibre exhibits an elongation at break that is 43% or below.

10. Film manufactured from a linear block polymer according to any of the claims 1-4.

11. Porous polymeric material manufactured from a linear block polymer according to any of the claims 1-4.

12. Implant for the implantation into the human or animal body, which implant comprises a linear block polymer according to any of the preceding claims.

## Patentansprüche

1. Lineares Block-Polymer gemäß Formel (I) 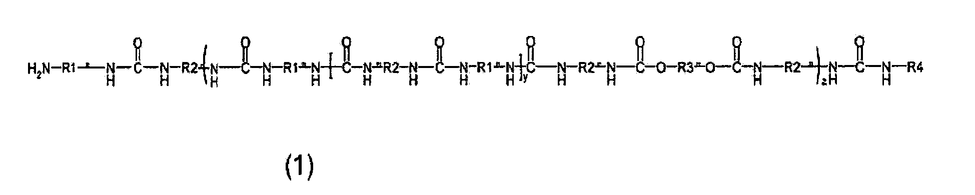 wobei
R1 von einem Diamin, z.B. Ethylendiamin, 1,2-Diaminopropan oder 1,3-Diaminopropan abgeleitet ist;
R2 von einem aromatischen Diisocyanat abgeleitet ist;
R3 von einem Esterdiol abgeleitet ist;
R4 von Dibutylamin oder Ethanolamin abgeleitet ist;
wobei 0 < y < 4 ist und z > 8 ist,
**dadurch gekennzeichnet, dass**
die Monomere, von denen R2 und R3 abgeleitet sind, in solchen Mengen hinzugegeben werden, dass das molare Verhältnis zwischen R2 und R3 größer als 2:1 ist.

2. Lineares Block-Polymer gemäß Anspruch 1, wobei R1 abgeleitet ist von Ethylendiamin, 1,3-Diaminopropan, 1,2-Diaminopropan, 1,4-Diaminobutan, 1,5-Diaminopentan oder 1,6-Diaminohexan.

3. Lineares Block-Polymer gemäß Anspruch 1 oder 2, wobei R3 von Polycaprolactondiol, Polydiethylenglycoladipat oder Poly(pentandiolpimelat) abgeleitet ist.

4. Lineares Block-Polymer gemäß einem der vorstehenden Ansprüche, wobei R2 von 4,4'-Diphenylmethandiisocyanat, Naphthalindiisocyanat oder Toluoldiisocyanat abgeleitet ist.

5. Faser, hergestellt aus einem linearen Block-Polymer gemäß einem der vorstehenden Ansprüche.

6. Faser gemäß Anspruch 5, wobei die Faser eine Zähigkeit von mindestens 0,1 N/Tex aufweist.

7. Faser gemäß Anspruch 6, wobei die Faser eine Zähigkeit von mehr als 0,2 N/Tex aufweist.

8. Faser gemäß einem der Ansprüche 5 bis 7, wobei die Faser eine Reißdehnung von weniger als 100 % aufweist.

9. Faser gemäß einem der Ansprüche 5 bis 7, wobei die Faser eine Reißdehnung von 43% oder weniger aufweist.

10. Faser, hergestellt aus einem linearen Block-Polymer gemäß einem der Ansprüche 1 bis 4.

11. Poröses Polymermaterial, hergestellt aus einem linearen Block-Polymer gemäß einem der Ansprüche 1 bis 4.

12. Implantat zum Implantieren in einen menschlichen Körper oder Tierkörper, wobei das Implantat ein lineares Block-Polymer gemäß einem der vorstehenden Ansprüche umfasst.

## Revendications

1. Polymère séquencé linéaire selon la formule (1) 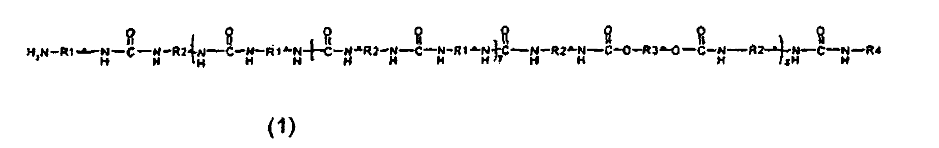 dans laquelle
R1 est dérivé d'une diamine, par exemple l'éthylène diamine, le 1,2-diamino propane ou le 1,3-diamino propane ;
R2 est dérivé d'un diisocyanate aromatique ;
R3 est dérivé d'un esterdiol ;
R4 est dérivé de dibutyl amine ou d'éthanolamine ;
où 0 < y < 4 et z > 8,
**caractérisé en ce que**,
les monomères dont R2 et R3 sont dérivés sont ajoutés en des quantités telles que le rapport molaire entre R2 et R3 est supérieur à 2 : 1.

2. Polymère séquencé linéaire selon la revendication 1, dans lequel R1 est dérivé de l'éthylène diamine, du 1,3-diamino propane, du 1,2-diamino propane, du 1,4-diamino butane, du 1,5-diamino pentane ou du 1,6-diamino hexane.

3. Polymère séquencé linéaire selon la revendication 1 ou 2, dans lequel R3 est dérivé du poly(caprolactone diol), du poly(adipate de diéthylène glycol) ou du poly(pimélate de pentane diol).

4. Polymère séquencé linéaire selon l'une quelconque des revendications précédentes, dans lequel R2 est dérivé du 4,4' diphényl méthane diisocyanate, du naphtalène diisocyanate ou du toluène diisocyanate.

5. Fibre fabriquée à partir d'un polymère séquencé linéaire selon l'une quelconque des revendications précédentes.

6. Fibre selon la revendication 5, laquelle fibre présente une ténacité d'au moins 0,1 N/Tex.

7. Fibre selon la revendication 6, laquelle fibre présente une ténacité au-dessus de 0,2 N/Tex.

8. Fibre selon l'une quelconque des revendications 5 à 7, laquelle fibre présente un allongement à la rupture qui est en dessous de 100 %.

9. Fibre selon l'une quelconque des revendications 5 à 7, laquelle fibre présente un allongement à la rupture qui est de 43 % ou en dessous.

10. Film fabriqué à partir d'un polymère séquencé linéaire selon l'une quelconque des revendications 1 à 4.

11. Matériau polymère poreux fabriqué à partir d'un polymère séquencé linéaire selon l'une quelconque des revendications 1 à 4.

12. Implant destiné à l'implantation dans le corps humain ou d'un animal, lequel implant comprend un polymère séquencé linéaire selon l'une quelconque des revendications précédentes.
